# EUROPEAN PATENT APPLICATION

(11) **EP 4 477 663 A1**
(43) Date of publication of application: **18.12.2024**
(21) Application number: 23179180.7
(22) Date of filing: 14.06.2023
(51) Int. Cl.: C07K 14/195, C07K 14/005, C07K 14/245, C07K 14/25, C12N 15/32, C12N 15/62, C12Q 1/6844

(54) **PROTEINS FOR ISOTHERMAL AMPLIFICATION**

(71) Applicant: midge medical GmbH, 12099 Berlin (DE)
(72) Inventor: Weidmann, Manfred, 12099 Berlin (DE)
(74) Representative: Eisenführ Speiser

(57) **Abstract**

The present invention relates to the field of isothermal amplification of nucleic acids, including DNA and RNA. In particular, the invention relates to novel proteins for isothermal amplification as well as kit and methods comprising one or more of the proteins of the invention for the amplification of target nucleic acids, in particular for diagnostic detection, in a laboratory as well as a non-laboratory environment.

## Description

### Technical field

The present invention relates to the field of isothermal amplification of nucleic acids, including DNA and RNA. In particular, the invention relates to novel proteins for isothermal amplification as well as kit and methods comprising one or more of the proteins of the invention for the amplification of target nucleic acids, in particular for diagnostic detection, in a laboratory as well as a non-laboratory environment.

### Background

Nucleic acid amplification techniques, including detection techniques, are broadly used in research and medical diagnostics. Such techniques, like molecular real-time PCR assays, are usually very sensitive, and offer target specificity but they still suffer from certain drawbacks. In a PCR the primer annealing can only occur through repetitive cycling between high temperatures allowing thermal denaturing of double-stranded DNA (dsDNA) and lower temperatures that allow annealing of primers, and primer extension by the polymerase. The inherent need for ongoing cycles of rapid cooling and heating (twenty to fifty cycles of two to three alternating temperatures), accurate temperature control, and the use of high (>90°C) temperatures requires specialized thermal cyclers and is highly energy-consuming. Thus, PCR-based methods for DNA amplification have the huge disadvantage to require highly equipped laboratories and well-trained personnel. Further, the highest possible efficiency for PCR methods is inevitably limited to one doubling per cycle, which does not allow quick (10-20 min) amplification while maintaining a high yield. The outbreak of SARS-CoV-2 has shown that PCR-based diagnosis by specialized laboratories is too slow and cumbersome for a responsive monitoring of infectious events. Thus, it is still impossible to react quickly to local virus outbreaks and to stop the viral spread effectively. It was tried to meet the evident requirement for fast and easy testing and point-of-need diagnosis by antigen-based diagnostic tools. However, the detection of viral RNA or DNA, which is critical for the accurate diagnosis of a viral infection, remains to be slow and cumbersome. Therefore, new portable diagnostic solutions having a high sensitivity and specificity and being able to provide reliable results at the place of testing are urgently needed.

A solution that allows both quick point-of-need diagnosis and the detection of viral RNA or DNA might be PCR alternatives, the so-called isothermal amplification methods (for a review, see Zanoli and Spoto, Biosensors (Basel), 2013 3(1): 18-43) or Glöckner et al., Critical Reviews in Biochemistry and Molecular Biology, 2021 56:6, 543-586). The main advantage over PCR is the fact that isothermal nucleic acid amplification methods do not require any thermal cycling, but can rather be conducted at constant temperatures. This makes the amplification process much easier to operate and to control and less energy-consuming than PCR methods. This is in particular advantageous as testing devices can be constructed with fewer technical requirements, which is necessary to provide cost-efficient yet sensitive point of care testing. The constant temperature of isothermal methods additionally allows the use of fully enclosed micro-structured devices, which reduces the risk of sample contamination and implies low sample consumption, multiplex DNA analysis, and portable devices realization. Finally, the constant temperature would be highly preferably for point-of-need and/or portable diagnostic devices, as recently developed by the present applicant (DE 10 2020 109 744.1). As clearly exemplified during the Corona pandemic in 2020 - 2022, there is still an ongoing global need for providing fast and reliable nucleic acid amplification techniques, which cannot exclusively be performed in specialized diagnostic centers, but can rather be performed immediately at the site, where a biological sample is obtained from a person to be tested. Further, it has been observed at a global scale that time to result is of paramount importance as, unless strict and prophylactic quarantine rules apply, there is a huge risk that an infected yet symptomless patient may spread an infectious disease until receiving a (reliable) test result, which can currently last anything from 24-36 hours or more.

There are numerous isothermal amplification strategies available at date, including nucleic acid sequence-based amplification (NASBA), loop-mediated isothermal amplification (LAMP), helicase-dependent amplification (HDA), rolling circle amplification (RCA), multiple displacement amplification (MDA), recombinase polymerase amplification (RPA), and nicking enzyme amplification reaction (NEAR/EXPAR) (see: Zanoli and Spoto, 2013, Biosensors (Basel). 2013 Mar; 3(1): 18-43; for a comprehensive survey on nowadays RPA techniques: Li et al., Analyst, 2019, 144, 31, pp. 31 to 67, or strand invasion based amplification (SIBA^{®}- SIBA in the following) Hoser et al., 2014 PLoS ONE 9(11): e112656, or NEAR/EXPAR: Van Ness et al., 2003, PNAS April 15, 2003 100 (8) 4504-4509). What the various isothermal amplification strategies have in common is that they all offer particular methods for allowing the initiation of the polymerase reaction. In conventional PCR this is achieved through the thermal cycling, which allows the primer to anneal to single stranded DNA (ssDNA) after denaturing.

A typical RPA system for RNA amplification and detection requires the staggering number of 7 different proteins: a recombinase, a recombinase-assisting factor, a single-stranded DNA-binding protein, a strand displacing polymerase, an energy regeneration system, an exonuclease and a reverse transcriptase. This renders it difficult to produce cost-efficient RPA kits and compositions for mass use. Moreover, for POC (point of care) diagnostics and similar ready-to-use applications it is necessary, or at least highly desirable, to provide all enzymes as dry pellets that can be easily reconstituted in a suitable buffer when used. While pelleting and reconstitution of enzymes, in particular for complex enzyme mixtures, can generally be a challenging task, it is especially critical for POC systems, which need to allow an immediate, robust, effective and reliable reaction, while ideally also being cost-efficient.

RPA amplification and the involved components have been optimized extensively in the past, yet the yet the involved enzyme are usually not exchanged during attempt to improve the amplification, rather relying on stepwise modifications to the established factors. This leaves a vast untapped and mostly unexplored potential of completely novel factors that could help to drastically improve isothermal amplification.

It was thus an object of the invention to identify and provide completely novel proteins from different species to advance the currently available possibilities for isothermal amplification. Technically, it is very complex to exchange enzymes of a known system, especially for a complex RPA system, which requires an intricate interplay of multiple different proteins in context of the overall reaction conditions. Moreover, it is a highly challenging task to identify novel candidates among the myriad of possible candidate proteins and hypothetical proteins known only from genetic screens. The endeavor to identify and test completely new proteins, instead of trying to optimize the proteins that are already in use, requires extensive efforts in screening, verifying and testing and involves having to discarding multiple suboptimal candidates along the way. Despite these challenges, it is an important task and therefore and object of the present invention to optimize available RPA methods, in particular for diagnostic amplification methods, to create a more powerful but cost-efficient diagnostic tool, in particular to provide cheap, but highly reliable and fast POC testing tools.

### Summary of the Invention

The above objects have been achieved by providing novel proteins allowing amplifications reactions, including RPA, that can be performed in a more economic way requiring less enzymes and/or being efficient enough to be so that the methods can be performed without ATP or an ATP analog and/or an energy regeneration system and/or without additional crowding agents, as the novel enzymes are much more efficient,

In one aspect, there is provided a protein, or a functional fragment thereof, preferably supporting isothermal amplification, wherein the protein comprises or consists of: a) a *Lutimaribacter* SSB1 comprising or consisting of the amino acid sequence of SEQ ID NO: 1, or a sequence having at least 90%, 91%, 92%, 93%, 94%, 95%, 96%, 97%, 98% or at least 99% sequence identity thereto, preferably wherein the amino acid sequence has a serine residue at position 64 according to SEQ ID NO 1 or 2; or b) a *Lutimaribacter* SSB2 comprising or consisting of the amino acid sequence of SEQ ID NO: 2, or a sequence having at least 90%, 91 %, 92%, 93%, 94%, 95%, 96%, 97%, 98% or at least 99% sequence identity thereto; or c) a *Shigella flexneri* SSB comprising or consisting of the amino acid sequence of SEQ ID NO: 3, or a sequence having at least 90%, 91%, 92%, 93%, 94%, 95%, 96%, 97%, 98% or at least 99% sequence identity thereto; or d) an Bacteriophage λ Beta protein comprising or consisting of the amino acid sequence of SEQ ID NO: 4, or a sequence having at least 90%, 91%, 92%, 93%, 94%, 95%, 96%, 97%, 98% or at least 99% sequence identity thereto; or e) a *Lutimaribacter* RecA1 comprising or consisting of the amino acid sequence of SEQ ID NO: 5 or 6, or a sequence having at least 90%, 91%, 92%, 93%, 94%, 95%, 96%, 97%, 98% or at least 99% sequence identity thereto; or f) a *Lutimaribacter* RecA2 comprising or consisting of the amino acid sequence of SEQ ID NO: 7, or a sequence having at least 90%, 91%, 92%, 93%, 94%, 95%, 96%, 97%, 98% or at least 99% sequence identity thereto; or g) a *Shigella flexneri* RecA comprising or consisting of the amino acid sequence of SEQ ID NO: 8, or a sequence having at least 90%, 91%, 92%, 93%, 94%, 95%, 96%, 97%, 98% or at least 99% sequence identity thereto; or h) a *Escherichia coli* RecT comprising or consisting of the amino acid sequence of SEQ ID NO: 9, or a sequence having at least 90%, 91%, 92%, 93%, 94%, 95%, 96%, 97%, 98% or at least 99% sequence identity thereto; or i) a Bacteriophage λ Orf comprising or consisting of the amino acid sequence of SEQ ID NO: 10, or a sequence having at least 90%, 91 %, 92%, 93%, 94%, 95%, 96%, 97%, 98% or at least 99% sequence identity thereto.

In one embodiment, there is provided a fusion protein comprising at least one protein of the first aspect and at least one further functional portion, the at least two portions optionally being separated by at least one linker.

Further provided in a second aspect is a fusion protein, wherein the fusion protein comprises at least one affinity tag, preferably at the C-terminus and/or N-terminus.

Yet further provided is a fusion protein of the above aspects and embodiments, wherein the fusion protein comprises at least one GST-tag, MBP-tag, Strep-tag and/or HIS-tag, preferably comprising at least one HIS-tag.

In a third aspect, a nucleic acid molecule encoding at least protein of the first aspect; and/or at least one fusion protein of the second aspect is provided.

In a fourth aspect, there is provided a vector or expression construct comprising at least one nucleic acid molecule of the fourth aspect.

In a fifth aspect, there is provided a cell comprising at least one protein of the first aspect; and/or at least one fusion protein of the second aspect; and/or at least one nucleic acid molecule of the third aspect and/or at least one vector or expression construct of the fourth aspect.

In a sixth aspect, there is provided a kit comprising at least one protein of the first aspect; and/or at least one fusion protein of the second aspect; and/or at least one nucleic acid molecule of the third aspect; and/or at least one vector or expression construct of the fourth aspect; and/or at least one cell of the fifth aspect. In one embodiment, kit comprises (i) at least one single-stranded binding protein, optionally of the first aspect item a), b), c) and/or d), preferably of the first aspect item a), and/or at least fusion protein of the second aspect comprising the same; (ii) at least on recombinase, optionally of aspect 1 item e), f), g) and/or h), and/or at least fusion protein of the second aspect comprising the same; (iii) optionally at least one recombinase-assisting factor, optionally of the first aspect item i), and/or at least fusion protein of the above second aspect comprising the same; (iv) at least one, preferably at least two, target sequence specific primers; (v) at least one DNA polymerase; (vi) optionally at least one reverse transcriptase; (vii) suitable reaction components, including at least one suitable buffer, dNTPs or a mixture of dNTPs and ddNTPs, optionally ATP or an ATP analog, and further optionally a crowding agent; (viii) optionally at least one probe and further optionally at least one enzyme activating the probe.

In another aspect, there is provided a use of protein of the first aspect; and/or a fusion protein of the second aspect; and/or a nucleic acid molecule of the third aspect; and/or a vector or expression construct of the fourth aspect; a cell of the fifth aspect, and/or a kit of the sixth aspect for isothermal amplification of at least one target nucleic acid molecule, optionally for detection of viral RNA and/or DNA, and/or for sample amplification for nucleic acid sequencing, preferably wherein the amplification is performed *in vitro* and/or preferably, wherein the amplification is performed as *in situ* available test, preferably using a kit of sixth aspect.

In yet another aspect, there is provided an isothermal method of nucleic acid amplification of at least one target sequence, preferably in a biological sample, the method comprising the steps of: (a) providing (i) at least one single-stranded binding protein, optionally of the first aspect item a), b), c) and/or d), preferably of the first aspect item a), and/or at least fusion protein of the second aspect comprising the same; (ii) at least on recombinase, optionally of the first aspect item e), f), g) and/or h), and/or at least fusion protein of the second aspect comprising the same; (iii) optionally at least one recombinase-assisting factor, optionally of the first aspect item i), and/or at least fusion protein of the second aspect comprising the same; (iv) at least one, preferably at least two, target sequence specific primers; (v) at least one DNA polymerase; (vi) optionally at least one reverse transcriptase; (vii) suitable reaction components, including at least one suitable buffer, dNTPs or a mixture of dNTPs and ddNTPs, optionally ATP or an ATP analog and further optionally a crowding agent; (b) providing at least one biological sample to be analyzed for the presence of the at least one target sequence and optionally providing a preferably sterile extraction kit to obtain the biological sample; (c) optionally: providing at least one probe and optionally providing at least one enzyme activating the probe so that a detectable signal is generated; (d) adding a starting reagent to initiate the amplification reaction; and (e) obtaining the at least one amplified target sequence and/or obtaining at least one detectable signal each signal being indicative of a successful amplification of the respective target sequence amplified, wherein step (a) comprises in sub-step (i), (ii) and/or (ii) the provision of at least one protein of the first aspect item and/or at least one fusion protein of the second aspect.

In one embodiment there is provided a method, wherein the method does not comprise the use of ATP or an ATP analog and/or an energy regeneration system.

In yet another embodiment, there is provided a method, wherein the method is performed absent a recombinase-assisting factor.

### Brief Description of Drawings

**Figure 1A** (**Fig. 1A**) Shows the purification of LuSSB1. 1: soluble fraction, 2: insoluble fraction, 3 and 4: flow-through, 5 to 8: wash fractions, 9 to 14: elution fraction, L: unstained protein standard (10-20 kDa, NEB)
**Figure 1B** (**Fig. 1B**) Shows the purification of LuSSB2. 1: soluble fraction, 2: insoluble fraction, 3: flow-through, 4: wash fraction, 5 to 7: elution fraction, L: unstained protein standard (10-20 kDa, NEB)
**Figure 1C** (**Fig. 1C**) Shows the purification of SfRecA. 1: soluble fraction, 2: insoluble fraction, 3: flow-through, 4: wash fraction, 5 to 7: elution fraction, L: unstained protein standard (10-20 kDa, NEB)
**Figure 2A** (**Fig. 2A**) Shows an electrophoretic mobility shift assay (EMSA) with LuSSB2. Numbers indicate the amount of added LuSSB2 protein. "Oligo" denotes a negative control without added protein.
**Figure 2B** (**Fig. 2B**) Shows an electrophoretic mobility shift assay (EMSA) with SfSSB. Numbers indicate the amount of added SfSSB protein. "Oligo" denotes a negative control without added protein.
**Figure 2C** (**Fig. 2C**) Shows an electrophoretic mobility shift assay (EMSA) with LuSSB1. Numbers indicate the amount of added LuSSB1 protein. "Oligo" denotes a negative control without added protein.
**Figure 2D** (**Fig. 2D**) Shows an electrophoretic mobility shift assay (EMSA) with RecT. Numbers indicate the amount of added RecT protein. "Oligo" denotes a negative control without added protein.
**Figure 2E** (**Fig. 2E**) Shows an electrophoretic mobility shift assay (EMSA) with Beta Protein. Numbers indicate the amount of added Beta protein. "Oligo" denotes a negative control without added protein.
**Figure 2F** (**Fig. 2F**) Shows an electrophoretic mobility shift assay (EMSA) with AOrf. Numbers indicate the amount of added λOrf protein. "Oligo" denotes a negative control without added protein.
**Figure 3** (**Fig. 3**) Shows exemplary results of RPA reactions. "a" denotes an RPA mixture with LuSSB1 used as single-stranded binding protein. "b" denotes an RPA mixture with RB69 GP32 used as single-stranded binding protein. Additional curves show negative controls. Y-axis shows relative fluorescence units (Axxin T8 device) and x-axis shows time in seconds.
**Figure 4** (**Fig. 4**) Shows exemplary results of RPA reactions. "a" and "b" denote an RPA mixture with 330 ng/µl LuSSB1 used as single-stranded binding protein. "c" and "d" denote an RPA mixture with 580 ng/µl RB69 GP32 used as single-stranded binding protein. Y-axis shows relative fluorescence units (Axxin T8 device) and x-axis shows time in seconds.
**Figure 5** (**Fig. 5**) Shows exemplary results of an RPA reactions with symmetric and asymmetric primer concentrations. Circles represent reaction mix 1 with symmetric primer concentrations. Squares represent reaction mix 1 with 1:4 asymmetric primer concentrations. Triangles represent reaction mix 2 with 1:4 asymmetric primer concentrations. Y-axis shows tt (time threshold) values in seconds and x-axis shows number of DNA target molecules.

### Brief description of sequences

- **Seq ID NO: 1**: Amino acid sequence of *Lutimaribacter* sp. EGI FJ00015 singlestranded binding protein 1 (LuSSB1)
- **Seq ID NO: 2**: Amino acid sequence of *Lutimaribacter* sp. EGI FJ00015 singlestranded binding protein 2 (LuSSB2)
- **Seq ID NO: 3**: Amino acid sequence of *Shigella flexneri* single-stranded binding protein (SfSSB)
- **Seq ID NO: 4**: Amino acid sequence of Bacteriophage A Beta protein
- **Seq ID NO: 5**: Amino acid sequence of *Lutimaribacter* sp. EGI FJ00015 RecA1 (LuRecA1)
- **Seq ID NO: 6**: Amino acid sequence of *Lutimaribacter* sp. EGI FJ00015 RecA1 (LuRecA1) H64S
- **Seq ID NO: 7**: Amino acid sequence of *Lutimaribacter* sp. EGI FJ00015 RecA2 (LuRecA2)
- **Seq ID NO: 8**: Amino acid sequence of *Shigella flexneri* RecA (SfRecA)
- **Seq ID NO: 9**: Amino acid sequence of *Escherichia coli* RecT
- **Seq ID NO: 10**: Amino acid sequence of Bacteriophage λ Orf (AOrf)
- **Seq ID NO: 11**: Coding nucleic acid sequence of *Lutimaribacter* sp. EGI FJ00015 single-stranded binding protein 1 (LuSSB1)
- **Seq ID NO: 12**: Coding nucleic acid sequence of *Lutimaribacter* sp. EGI FJ00015 single-stranded binding protein 2 (LuSSB2)
- **Seq ID NO: 13**: Coding nucleic acid sequence of *Shigella flexneri* single-stranded binding protein (SfSSB)
- **Seq ID NO: 14**: Coding nucleic acid sequence of Bacteriophage A Beta protein
- **Seq ID NO: 15**: Coding nucleic acid sequence of *Lutimaribacter* sp. EGI FJ00015 RecA1 (LuRecA1)
- **Seq ID NO: 16**: Coding nucleic acid sequence of *Lutimaribacter* sp. EGI FJ00015 RecA1 (LuRecA1) H64S
- **Seq ID NO: 17**: Coding nucleic acid sequence of *Lutimaribacter* sp. EGI FJ00015 RecA2 (LuRecA2)
- **Seq ID NO: 18**: Coding nucleic acid sequence of *Shigella flexneri* RecA (SfRecA)
- **Seq ID NO: 19**: Coding nucleic acid sequence of *Escherichia coli* RecT
- **Seq ID NO: 20**: Coding nucleic acid sequence of Bacteriophage A Orf (AOrf)

A "biological sample" is to be understood broadly and relates to any material isolated from a living organism, which contains genetic material from this organism and/or (additionally) from a pathogen (a pathogenic prokaryote or eukaryote) or virus (with an RNA or a DNA genome, single, or double-stranded, or a retrovirus) having infected or infiltrated the organism, or living in symbiosis with this organism etc. A biological sample includes, for example, swab and saliva samples, including nasopharyngeal and oropharyngeal swab or saliva, but also sputum, blood, urine and stool and the like. Preferably, a biological sample to be tested in a non-laboratory environment will be a swab, saliva, urine, or stool sample etc. that can be retrieved without the assistance of trained medical personnel.

A "crowding agent" as used herein is meant to specify any agent assisting in providing an optimum reaction environment for an enzymatic reaction. The mode of action of a crowding agent is the creation of smaller reaction compartments so that the probability of a reaction between a substrate and the cognate enzyme is increased. Crowding agents usually actively added to isothermal amplification reactions include, for example, polyethylene glycol (PEG), dextran and ficoll. The crowding agent may usually be at a concentration between 1% and 12% by weight (w/v) of the reaction. In preferred embodiments, a crowding agent is used at a concentration of less than 5% by weight (w/v) as disclosed herein. While all PEG polymers are useful, preferred PEG compounds were disclosed to include PEG1450, PEG3000, PEG8000, PEG10000, PEG compound molecular weight 15000-to 20,000 (also known as Carbowax 20M), and a combination thereof.

Whenever the present disclosure relates to the percentage of the identity of nucleic acid or amino acid sequences, this identity is determined by a comparison of a sequence of interest, the reference sequence (e.g., a SEQ ID NO as disclosed herein), to another sequence, the query sequence, over the entire length of the reference sequence. Identity is obtained by using the EMBOSS Water Pairwise Sequence Alignments (nucleotide) program or the EMBOSS Water Pairwise Sequence Alignments (protein) program (www.ebi.ac.uk/Tools/psa/emboss_water/) for amino acid sequences. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

A "label" can be a chemical modification or isotope substitution that enables the identification of a molecule, moiety or atom throughout its metabolic transformations or transport, or particularly during the course of a diagnostic or preparative assay of nucleic acid amplification.

The term "probe" as used herein broadly refers to a molecule or atom used in molecular biology, and particularly in the methods as disclosed herein, to study the properties or, for diagnostic setting, the quantity of another molecule or structure of interest. A variety of different probes that can be easily combined with the methods as disclosed herein is available to the skilled person (cf. Molecular Probe Techniques, Tree Number(s) E05.601, Unique ID D015336, RDF Unique Identifier, http://id.nlm.nih.gov/mesh/D015336). For using the methods as disclosed herein on the specific device as disclosed herein, probes, optionally activatable probes, providing a detectable and/or quantifiable signal may be preferred, e.g. Förster/fluorescence resonance energy transfer (FRET) probes etc. In view of the methods as disclosed herein, this may allow a rapid diagnostic result. This offers the advantage to include a positive control associated with one probe, and a different probe associated with the actual target sequence, which may drastically increase the reliability of a diagnostic result.

A single protein as used herein refers to a protein that consists of one continuous polypeptide chain, or two or more subunits that interact closely, such as a homo- or hetero-dimer, wherein the single protein can have more than one distinct function.

A "functional portion" as used herein describes a part of a protein that can, without the rest the protein, fulfill at least one desired function of the whole-length protein. For example, a functional portion of an SSB protein is a part of said SSB protein that has single-stranded (DNA) binding activity, when present without the remaining part(s) of said whole-length SSB protein.

A "fusion protein" as used herein refers to a continuous polypeptide chain or two polypeptide chains linked by a non-peptide linker of two amino acid sequences that naturally do not occur in this juxtaposition. Preferably, a fusion protein is a continuous polypeptide chain, for example two amino acid sequences linked by a peptide linker. Commonly, a fusion protein is produced by expressing a DNA sequence comprising an ORF encoding the fused sequence. Design and expression and/or production of fusion proteins are well known to the skilled person.

A reverse transcriptase may be any enzyme that can generate a complementary DNA (cDNA) strand from a template RNA molecule. "Functionality" in the context of the present invention refers to the ability perform the function of a certain enzyme in light of the present invention. Thus, a reverse transcriptase functionality refers to the ability of to generate cDNA from a target RNA in the reaction mix and DNA polymerase functionality refers to the ability to polymerize DNA in on a target DNA strand in the reaction mix.

A "single-strand binding protein" (also called single-stranded (DNA-)binding protein) or "SSB" or "SSB protein" as used herein, relates to proteins of the class of single-strand binding protein (e.g. reviewed in James L. Keck (ed.), Single-Stranded DNA Binding Proteins: Methods and Protocols, Methods in Molecular Biology, vol. 922, DOI 10.1007/978-1-62703-032-8_1, # Springer Science+Business Media, LLC 2012).

The term "tag" or "protein tag" refers to a variety of different polypeptide sequences or the corresponding nucleic acid sequences encoding the same, which can be incorporated into a protein of interest at various positions, preferably at the N- and/or C-terminus, or between distinct functional domains, and fulfil several different functions. Protein tags can be, for example, affinity tags, such as chitin binding protein (CBP), maltose binding protein (MBP), Strep-tag and glutathione-S-transferase (GST) or His-Tag. Affinity tags can be used for specifically purifying the protein of interest. Another example of protein tags are solubilization tags, which allow to obtain the protein of interest in its soluble form especially in bacterial cell culture. Epitope tags can be used for having a binding site for analytical antibodies. Luminescence, preferably fluorescence tags offer the possibility to detect the protein of interest in the cell culture. A tag or label can also serve the purpose of labelling a protein or enzyme of interest, or a fragment thereof, to allow an easy identification. This may be of particular interest for diagnostic purposes.

### Detailed Description

Even though nucleic acid amplification techniques, including isothermal amplification strategies, have made enormous progress over the last decades, the crucial working enzymes for all of these techniques are usually not exchanged, or they are slightly modified in a stepwise manner.

RPA amplification methods of the prior art usually starts with the binding of a phage-derived T4 UvsX protein as central recombinase, assisted by the T4 UvsY acting as loading factor, to the primers. This complex invades the double-stranded (ds) DNA, forming a so-called D-loop in which the primer hybridizes with the template strand, initiating a strand exchange reaction. The unwound complementary strand is stabilized by a single-strand binding (SSB) protein, where usually a T4-derived gp32 protein is used. Primer incorporation allows a strand displacing DNA polymerase, usually a *Bsu* (*Bacillus subtilis*-derived) or *Sau* (*Staphylococcus aureus*-derived) polymerase, to initiate the synthesis from the free 3'-OH of the primer. As the polymerization continues, the two parental strands will thus continue to separate. Incorporation of both forward and reverse primers then enables strand synthesis to occur in both directions simultaneously, and ultimately results in the exponential accumulation of amplified duplex DNA, consisting of the sequence between the forward and reverse primers. Consequently, standard RPA requires a significant amount of protein/enzyme interaction partners to guarantee efficient DNA/RNA amplification, which seems to explain a reluctance to exchange involved proteins.

Extensive sequence-based screens as well as structural analyses and experimental assays as described in the examples below, lead to the identification of several bacterial recombinase and SSB proteins. Among these are *Lutimaribacter* SSB1 (LuSSB1), *Lutimaribacter* SSB2, (LuSSB2), Shigella flexneri RecA (SfRecA), Lutimaribacter RecA1 (LuRecA1), Lutimaribacter RecA2 (LuRecA2) and Shigella flexneri SSB (SfSSB).

Phage Recombinase enzymes can be subdivided into two groups: (i) UvsX and Gp2.5 are found ing virulat phages and (ii) Sak, Beta, Erf and Sak4 are found in temperate or formerly temperate phages (for an overview over phage recombinases see: (Lopes et al. 2010; doi: 10.1093/nar/gkq096. Epub 2010 Mar 1. PMID: 20194117; PMCID: PMC2896510). The respective classes can be characterized as follows: UvsX, is a RecA-like enzyme found in phage T4, among others, and has orthologs in other phages with large genomes and strictly virulent lifestyles. Gp2.5 recombinase is encoded by virulent phage T7, among others, and has functional and structural homology with SSB proteins. However, unlike SSB, it also possesses a recombinase function. Sak, Beta, and Erf, which have been delineated as three distinct families based on their primary sequences, possess single-stranded annealing activity. Three striking features distinguish these recombinases from RecA-like enzymes: (i) their ATP independence, (ii) their high efficiency in pairing short, 40-50 bp long sequences (30), and (iii) their ability to incorporate short single-stranded DNA substrates into the bacterial chromosome. Beta originates from the lambda phage and is part of the recombination system, which also consists of the Exo, Gam, and λOrf (or NinB) proteins. Beta appears to exhibit both SSB activity and recombinase activity. In general, Beta works together with AOrf, which is a functional equivalent of UvsY. It has been described previously that λOrf can substitute for RecFOR in the process of recombination (Maxwell et al., PNAS 2005 Aug 9;102(32):11260-5). However, this seems to be true only for λ- recombination. This seems to be related to the fact that λOrf can only efficiently displace Beta from nucleic acid, but not bacterial SSBs. Thus, RecA from bacteria may be combined with λOrf and Beta for RPA methods. This would have the advantage of dispensing with the bacterial helper proteins RecFOR. Moreover, since Beta exhibits recombinase activity, RPA may be able to function without RecA. A system that is very similar to the Red recombinase system is the RecT/E system. An particular advantage advantage of both Beta/Orf and RecT is that they work independently of ATP (Li et al., J Mol Biol. 1998 Mar 6;276(4):733-44;Rybalchenko et al., PNAS 2004 Dec 7;101(49):17056-60; Noirot and Kolodner, . J Biol Chem. 1998 May 15;273(20):12274-80). Based on these properties, Beta, λOrf and RecT were also further selected for synthesis and experimental evaluation of the proteins.

An important feature of the present invention is its ability to reliably amplify nucleic acid sequences of any kind, particularly also including linear target molecules. For a method that is to be used as a diagnostic tool for viral infections, the suitability of the method to detect linear targets is essential. Different viruses contain genetic material in various different forms as linear or circular, double-stranded or single-stranded DNA or RNA. Thus, an isothermal amplification method for detecting viral nucleic acid material needs to be able to detect all possible various forms viral nucleic acid material. Detection of linear nucleic acid targets is necessary for viruses with linear DNA but also for all RNA viruses as the RNA has to be reverse transcribed into linear complementary DNA (cDNA). Further, the detection of linear nucleic acid targets is required to detect RNA transcripts, also from DNA viruses.

In a first aspect, there is provided a protein, or a functional fragment thereof, preferably supporting isothermal amplification, wherein the protein comprises or consists of a) a *Lutimaribacter* SSB1 comprising or consisting of the amino acid sequence of SEQ ID NO: 1, or a sequence having at least 90%, 91%, 92%, 93%, 94%, 95%, 96%, 97%, 98% or at least 99% sequence identity thereto, preferably wherein the amino acid sequence has a serine residue at position 64 according to SEQ ID NO 1 or 2; or b) a *Lutimaribacter* SSB2 comprising or consisting of the amino acid sequence of SEQ ID NO: 2, or a sequence having at least 90%, 91%, 92%, 93%, 94%, 95%, 96%, 97%, 98% or at least 99% sequence identity thereto; or c) a *Shigella flexneri* SSB comprising or consisting of the amino acid sequence of SEQ ID NO: 3, or a sequence having at least 90%, 91%, 92%, 93%, 94%, 95%, 96%, 97%, 98% or at least 99% sequence identity thereto; or d) an Bacteriophage A Beta-protein comprising or consisting of the amino acid sequence of SEQ ID NO: 4, or a sequence having at least 90%, 91%, 92%, 93%, 94%, 95%, 96%, 97%, 98% or at least 99% sequence identity thereto; or e) a *Lutimaribacter* RecA1 comprising or consisting of the amino acid sequence of SEQ ID NO: 5 or 6, or a sequence having at least 90%, 91%, 92%, 93%, 94%, 95%, 96%, 97%, 98% or at least 99% sequence identity thereto; or f) a *Lutimaribacter* RecA2 comprising or consisting of the amino acid sequence of SEQ ID NO: 7, or a sequence having at least 90%, 91%, 92%, 93%, 94%, 95%, 96%, 97%, 98% or at least 99% sequence identity thereto; or g) a *Shigella flexneri* RecA comprising or consisting of the amino acid sequence of SEQ ID NO: 8, or a sequence having at least 90%, 91%, 92%, 93%, 94%, 95%, 96%, 97%, 98% or at least 99% sequence identity thereto; or h) a *Escherichia coli* RecT comprising or consisting of the amino acid sequence of SEQ ID NO: 9, or a sequence having at least 90%, 91%, 92%, 93%, 94%, 95%, 96%, 97%, 98% or at least 99% sequence identity thereto; or i) a Bacteriophage A Orf comprising or consisting of the amino acid sequence of SEQ ID NO: 10, or a sequence having at least 90%, 91%, 92%, 93%, 94%, 95%, 96%, 97%, 98% or at least 99% sequence identity thereto.

Proteins of the invention may be expressed and purified by standard methods known in the art. An exemplary and non-limiting example of an expression and purification scheme is shown in example 2 below.

In a second aspect, there is provided a fusion protein comprising at least one protein of the first aspect and at least one further functional portion, the at least two portions optionally being separated by at least one linker.

A linker as used herein refers to a molecule, typically a stretch of polypeptide (linker peptides) that may be inserted between different parts of a protein, in particular a fusion protein. Typically, a linker is positioned between two different polypeptides that are fused together (via the linker) to form a fusion protein; for example, a tag fused to a protein. A peptide linker useful with this invention may be 1 to 100 or more amino acids in length. A linker may be a flexible linker, a rigid linker, and/or a cleavable linker. A peptide linker may, for example, be a GS linker, but any peptide linker described in the art, may be used with the present invention.

In one embodiment, the fusion protein comprises at least one affinity tag, preferably at the C-terminus and/or N-terminus.

In one embodiment, the fusion protein comprises at least one GST-tag, MBP-tag, Strep-tag and/or HIS-tag, preferably comprising at least one HIS-tag.

As it is well known to the skilled person, creation of a fusion protein, such as a tag fused to a protein, may require some testing of solubility, stability, activity and the like with different positions of the fused components (e.g. the fused tag) as well as different types and/or length of linkers to achieve optimal results. Methods of designing, expressing and testing fusion proteins are well known in the art and are readily available to the skilled person. Testing of activity of a protein to be used in isothermal amplification, may be performed as known in the art and/or as disclosed herein.

In one embodiment, the GST-tag, MBP-tag, Strep-tag and/or HIS-tag is fused to the fusion protein via a cleavable linker.

In a third aspect, there is provided a nucleic acid molecule encoding at least one protein of the first aspect; and/or at least one fusion protein of any one of claims second aspect.

In a fourth aspect, there is provided a vector or expression construct comprising at least one nucleic acid molecule of the third aspect.

The vector or expression construct of the fourth aspect may comprise regulatory sequences, including all further nucleic acid sequences necessary for expression and replication in a desired target cell, such as a promoter and terminator operably linked to the sequence to be expressed, an origin of replication, and/or a selection marker. The skilled person is well aware that the choice of regulatory sequences is dependent on the choice of target cell. Recombinant protein expression, as well as suitable vectors and expression systems, promoters and further regulatory sequences are well-established for a multitude of cell types.

The nucleic acid of the third aspect or the vector or expression construct of the forth aspect may comprise a cDNA or coding sequence, devoid of introns, encoding the acyltransferase and/or Fusion protein of the present invention, e.g. for expression in prokaryotic cells.

In one embodiment, the nucleic acid of the third aspect or the vector or expression construct of the forth aspect may comprise a sequence selected from SEQ ID NO: 11 to 20, or a sequence having 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% sequence identity thereto.

The nucleic acid sequence, comprised by the nucleic acid of the third aspect and the vector or expression construct of the fourth aspect, encoding the at least protein of the first aspect; and/or at least one fusion protein of any one of claims second aspect may be codon optimized for expression in the desired host cell.

In a fifth aspect, there is provided a cell comprising at least one protein of the first aspect; and/or at least one fusion protein of any one of the second aspect; and/or at least one nucleic acid molecule of the third aspect; and/or at least one vector or expression construct of the fourth aspect.

The cell may, for example, be a cell of a commonly used cellular expression system, including prokaryotic expression systems and eukaryotic expression systems.

In a sixth aspect, there is provided a kit comprising at least one protein of the first aspect; and/or at least one fusion protein of any one of the second aspect; and/or at least one nucleic acid molecule of the third aspect; and/or at least one vector or expression construct of the fourth aspect; and/or at least one cell of the fifth aspect.

In one embodiment, the kit comprises (i) at least one single-stranded binding protein, optionally of proteins a), b), c) and/or d) of the first aspect, preferably of protein a) of the first aspect, and/or at least fusion protein of the second aspect comprising the same; (ii) at least on recombinase, optionally of proteins e), f), g) and/or h) of the first aspect, and/or at least fusion protein of the second aspect comprising the same; (iii) optionally at least one recombinase-assisting factor, optionally of protein i) of the first aspect, and/or at least fusion protein the second aspect comprising the same; (iv) at least one, preferably at least two, target sequence specific primers; (v) at least one DNA polymerase; (vi) optionally at least one reverse transcriptase; (vii) suitable reaction components, including at least one suitable buffer, dNTPs or a mixture of dNTPs and ddNTPs, optionally ATP or an ATP analog and further optionally a crowding agent; (viii) optionally at least one probe and further optionally at least one enzyme activating the probe.

In one embodiment, the beta protein according to protein d) of the first aspect, or a fusion protein of the second aspect comprising the same, fulfills the function of the SSB protein of component (i) and the function of recombinase of component (ii). Thus, the beta protein according to protein d) of the first aspect, or a fusion protein of the second aspect comprising the same may simultaneously constitute components (i) and (ii) of the kit of the sixth aspect.

In embodiments in which component (i) and/or component (ii) of the kit of the eight aspect comprises or consists of the beta protein according to protein d) of the first aspect, or a fusion protein of the second aspect comprising the same, the recombinase-assisting factor of component (iii) preferably comprises or consist of λOrf of protein i) of the first aspect or a fusion protein of the second aspect comprising the same.

In one embodiment, component (i) and/or component (ii) of the kit of the sixth aspect comprises or consists of the beta protein according to protein d) of the first aspect, or a fusion protein of the second aspect comprising the same, and the kit does not comprise ATP or an ATP analog and/or does not comprise an energy regeneration system.

In one embodiment, component (ii) of the kit of the sixth aspect comprises or consists of RecT of according to protein h) of the first aspect, or a fusion protein of the second aspect comprising the same, and the kit does not comprise ATP or an ATP analog and/or does not comprise an energy regeneration system.

In one embodiment, the kit does not comprise a recombinase-assisting factor that is a protein.

It may be desirable to provide all proteins as pellets or powder, such as lyophilized pellets or powder to be reconstituted in the provided suitable buffer.

All reactions mixes disclosed herein may for example be provided in liquid form or as lyophilized pellets.

In preferred embodiments, the kit comprises an amount and/or concentration of crowding agent so that the concentration of crowding agent in the final reaction mixture is less than 5%, for example about 1%, about 2%, about 3%, about 3.5%, about 4% or about 4, 5%, wherein the concentration is by weight (w/v).

The crowding agent may, for example, be selected from the group consisting of polyethylene glycol (PEG), dextran, ficoll, PEG1450, PEG3000, PEG8000, PEG10000.

In certain embodiments, the kit does not comprise a crowding agent.

In one embodiment, the method does not comprise ATP or an ATP analog and/or an energy regeneration system; and/or wherein the method does not comprise a recombinase-assisting factor.

In one embodiment, the method comprises ATP or an ATP analog but does not comprise an energy regeneration system.

In embodiments relating to the amplification of RNA, the kit may comprises a reverse transcriptase. The kit may comprise a protein comprising both the functionality of a DNA polymerase and a reverse transcriptase so that one protein may be used to fulfill both functions.

In some embodiments, the kit may further comprise at least one reducing agent, preferably dithiothreitol (DTT). The DTT concentration may be between 1 mM and 50 mM.

In some embodiments, the kit may further comprise at least one RNase inhibitor.

In one embodiment, at least one forward and at least one reverse primer are provided as target sequence specific primers, wherein at least one of these primers, preferably the reverse primer, is provided in a higher amount and/or concentration in relation to the other primer, wherein the amount and/or concentration may, for example, be about 1.1, 1.2, 1.25, 1.5, 1.75, 1.3, 1.4, 1.5, 1.6, 1.7, 1.8, 1.9, 2, 2.1 to 2.5, 2.6 to 2.9, 3 to 4, 5 to 6, 7 to 8 or 9 to 10 times higher.

The kit may further comprise instructions for use.

In some embodiments, the kit comprising at least one component configured to be applied to a system for analyzing the probe (such a system is disclosed in European patent application EP20201885.9).

In some embodiments, the kit further comprises a second part comprising (i), (ii), optionally (iii), (iv), (v), optionally (vi), (vii) and optionally (viii), wherein the second part comprises a predetermined second target sequence acting as positive control and at least one, preferably at least two primers specific for the second target sequence, and preferably a second probe.

Further details to suitable reaction component, primer design and concentrations to be achieved in the final reaction mixture are disclosed under the eighth aspect and apply to the sixth aspect as well. Of course, concentrations of component comprised by kit as provided may differ from the concentrations in the final reaction mixture when the kit is used.

In a laboratory, the skilled molecular biologist or trained personnel can easily conduct the methods as disclosed herein, design primers and/or probes for target nucleic acids to be amplified, and suitable reaction conditions can be easily modified, if desired. Of course, it is a much bigger challenge to perform the method in a non-laboratory environment. Particularly in the latter situation, it may be favorable to provide a kit comprising all necessary components in ready-made sterile vials or tubes, so that the (bio-)chemical agents and components can easily be re-activated (for enzymes, for example, after freeze-drying) and the reaction can be started in a convenient manner.

In a seventh embodiment, there is provided a use of protein of the first aspect; and/or a fusion protein of the second aspect; and/or a nucleic acid molecule of the third aspect; and/or a vector or expression construct of the fourth aspect; a cell of the fifth aspect; and/or a kit of the sixth aspect for isothermal amplification of at least one target nucleic acid molecule.

In an eighth aspect, there is provided, an isothermal method of nucleic acid amplification of at least one target sequence, preferably in a biological sample, the method comprising the steps of: (a) providing (i) at least one single-stranded binding protein, optionally of proteins a), b), c) and/or d) of the first aspect, preferably of protein a) of the first aspect, and/or at least fusion protein of the second aspect comprising the same; (ii) at least on recombinase, optionally of proteins e), f), g) and/or h) of the first aspect, and/or at least fusion protein of the second aspect comprising the same; (iii) optionally at least one recombinase-assisting factor, optionally of protein i) of the first aspect, and/or at least fusion protein the second aspect comprising the same; (iv) at least one, preferably at least two, target sequence specific primers; (v) at least one DNA polymerase; (vi) optionally at least one reverse transcriptase; (vii) suitable reaction components, including at least one suitable buffer, dNTPs or a mixture of dNTPs and ddNTPs, optionally ATP or an ATP analog, and further optionally a crowding agent; (b) providing at least one biological sample to be analyzed for the presence of the at least one target sequence and optionally providing a preferably sterile extraction kit to obtain the biological sample; (c) optionally: providing at least one probe and optionally providing at least one enzyme activating the probe so that a detectable signal is generated; (d) adding a starting reagent to initiate the amplification reaction; and (e) obtaining the at least one amplified target sequence and/or obtaining at least one detectable signal each signal being indicative of a successful amplification of the respective target sequence amplified, wherein step (a) comprises in sub-step (i), (ii) and/or (ii) the provision of at least one protein of the first aspect and/or at least one fusion protein of the second aspect.

In one embodiment, the beta protein according to protein d) of the first aspect, or a fusion protein of the second aspect comprising the same, of the first aspect fulfills the function of the SSB protein of step (a) (i) and the function of recombinase of step (a)(ii). Thus, providing the beta protein according to protein d) of the first aspect, or a fusion protein of the second aspect comprising the same, may fulfil steps (a)(i) and (a)(ii) of the method of the eighth aspect.

In embodiments in which the beta protein according to protein d) of the first aspect, or a fusion protein of the second aspect comprising the same, is provided in step (a)(i) and/or (ii) of the method of the eight aspect, the optionally at least one recombinase-assisting factor of step (iii) is preferably λOrf of protein i) of the first aspect, or a fusion protein of the second aspect comprising the same.

In one embodiment, the beta protein according to protein d) of the first aspect, or a fusion protein of the second aspect comprising the same, is provided in step (a)(i) and/or (ii) of the method of the eight aspect and the method does not comprise the provision of ATP or an ATP analog and/or does not comprise an energy regeneration system.

In one embodiment, RecT of according to protein h) of the first aspect, or a fusion protein of the second aspect comprising the same, is provided in step (a)(ii) of the method of the eight aspect and the method does not comprise the addition of ATP or an ATP analog and/or does not comprise an energy regeneration system.

In one embodiment, the kit does not comprise a recombinase-assisting factor.

In preferred embodiments, the concentration of crowding agent is less than 5%, for example about 1%, about 2%, about 3%, about 3.5%, about 4% or about 4, 5%, wherein the concentration is by weight (w/v).

Typical concentrations of crowding agent for an isothermal reaction are above 5% by weight (w/v). However, such concentrations of a crowding agent, such as PEG, can falsify measurements of fluorescence as a means to detect amplification. Thus, systems based on fluorescent labels cannot reliably detect an amplifications, which would particularly problematic for diagnostic testing kits and systems. In contrast, at lower concentrations of crowding agent this problem is not observed. The present inventors found out that the proteins of the present invention, in particular LuSSB1, allow the reduction of the concentration of crowding agent while still enabling a fast and reliable amplification reaction. Thus, the proteins of the present invention offer the advantage that they solve the problem of the interference of the crowding agent with fluorescent measurements by allowing lower concentrations of crowding agent.

The crowding agent may, for example, be selected from the group consisting of polyethylene glycol (PEG), dextran, ficoll, PEG1450, PEG3000, PEG8000, PEG10000.

In certain embodiments, the method does not comprise a crowding agent.

In one embodiment, the method does not comprise ATP or an ATP analog and/or an energy regeneration system; and/or wherein the method does not comprise a recombinase-assisting factor.

In one embodiment, the method comprises ATP or an ATP analog but does not comprise an energy regeneration system.

In another embodiment, at least one energy regeneration system may be provided. A number of systems are available and are meanwhile routinely used for recycling energy in isothermal RPA reactions and similar reactions. One of those systems is the creatine kinase/phosphocreatine system, or chicken myokinase, which converts a molecule of AMP and one of ATP to two molecules of ADP. ADP is then converted to ATP using the creatine kinase/phosphocreatine system (Li et al., supra). For these enzymes, a phosphocreatine di(tris) salt or buffer may be suitable (1 mM to 100 mM).

Still, it was also found that providing a simple surplus of ATP will perfectly suffice to perform a reliable amplification reaction in a single vial in short time providing a reliable result. Therefore, particularly if a kit and/or a diagnostic handheld system of the present invention is used to perform the methods as disclosed herein it may be preferable to provide a surplus of ATP in a concentration above 2 mM, or above 5 mM, or above 7.5 mM or above 15 mM up to 100 mM. For preparative purposes (e.g., DNA synthesis), a higher concentration of ATP will be needed than for a diagnostic purpose, where a defined amount of a rather short fragment between two primers will be amplified. It was found that providing a high concentration of ATP will suffice to drive the amplification reaction successfully, which can be preferable for several reasons: (i) the reaction will be cheaper, as no additional enzymes will be needed, and (ii) the system will be less complex and thus more stable and (iii) robust in its use, which will be a particular advantage if used in a home environment and when performed nearly automatically, and not by a trained expert in a laboratory field

The DNA polymerase used in any aspect of the present invention may be a DNA polymerase having a strand displacing activity, i.e. in the polymerization reaction it is able to displace a DNA strand that is hybridized or partially hybridized to the DNA strand on which the polymerase is performing the polymerization reaction.

In certain embodiments of the methods of the present invention, at least one recombinase-assisting factor may additionally be provided, preferably before step (d). Such an enzyme or chemical fulfils the function of a co-enzyme of the recombinase and/or the chemical factor activates, stabilizes or otherwise enhances the activity of the recombinase. For RPA reactions, UvsY is commonly such a recombinase-assisting enzyme or loading factor helping to start nucleoprotein filament building, or to stabilize the nucleoprotein complex with recombinase any kind (Li et al, supra, or EP 1 759 012 A2).

In some embodiments, the at least one recombinase-assisting factor is λOrf as disclosed in the first aspect, or a fusion protein of the second aspect comprising the same.

Another recombinase-assisting enzyme of interest may be one that can promote efficient disassembly of recombinase/dsDNA complexes after DNA synthesis initiation. These auxiliary enzymes include those that are capable of stimulating 3' to 5' disassembly and those capable of supporting 5' to 3' disassembly. Further recombinase-assisting enzymes may include several polymerases that can displace RecA or an equivalent, preferably thermostable, recombinase in the 3' to 5' direction and can stimulate 3' to 5' disassembly of recombinase-dsDNA complexes. These DNA polymerases include preferably thermostable equivalents of E. coli PolV and homologous polymerase of other species. Other recombinase-assisting enzymes include a class of enzymes called helicases which can be used to promote the disassembly of a recombinase from dsDNA. These promote disassembly in both the 5' to 3' and 3' to 5' directions. Helicases are essential components of the recombination process in vivo and function to move the branch points of recombination intermediates from one place to another, to separate strands, and to disassemble and recycle components bound to DNA. Still further recombinase-assisting enzymes include E. coli RecG homologoues and preferably thermostable enzymes. RecG can stimulate disassembly of branch structures. In its natural environment, this enzyme functions to reverse replication forks at sites of DNA damage by unwinding both leading and lagging strands driving the replication fork back to generate a 4-way junction.

Suitable reaction components for enzymatic reactions and the concentration and compounding thereof, in case the enzyme and the reaction for which these suitable reaction components are intended is known, can easily be determined by the skilled person. Certain aspects of particular relevance for the methods of the present invention will be disclosed in detail in the following.

A nucleic acid amplification reaction will require dNTPs (nucleotide triphosphates), for example, dATP, dGTP, dCTP, and dTTP. In leading and lagging strand amplifications, ATP, GTP, CTP, and UTP may also be included for synthesis of RNA primers. In addition, ddNTPs (ddATP, ddTTP, ddGTP and ddGTP) may be used to generate fragment ladders. The dNTPs may be used at a concentration of between 1 µM to 200 mM of each NTP species. A mixture of dNTP and ddNTP may be used with ddNTP concentrations at 1/100 to 1/1000 of that of the dNTP (1µM to 200 mM). The use of UTP may be particularly suitable in case a carry-over prevention kit (e.g., Thermo Fisher using a so-called AmpErase) is used. In these embodiments, a suitable, usually recombinant, uracil N-glycosylase (UNG) can be used to prevent that an amplification product is again amplified in a subsequent reaction. To this end, the reaction can perform faster and false-positive results can be avoided.

In some embodiments, the method may further comprise at least one reducing agent, preferably dithiothreitol (DTT). The DTT concentration may be between 1 mM and 50 mM.

In some embodiments, the method may further comprise at least one RNase inhibitor.

The buffer solution in an isothermal method of the present invention may be a Tris-HCl buffer, a Tris-Acetate buffer, or a combination thereof, but other buffer systems are suitable as well. The buffers may be present at a concentration of between around 10 nm to 500 mM, which will depend on the buffer system used and the pH of the reaction to be achieved. The latter is influenced by the optimum activity of an enzyme of interest, as it is known to the skilled person.

Standard suitable reaction conditions and concentrations of agents for the isothermal methods as disclosed herein (e.g., enzymes, primers, probe, dNTPs, ATP, buffers, pH, etc.) are known to the skilled person and cancan be taken from, for example, Zanoli and Spoto, *supra,* Li et al., *supra,* Hoser et al., *supra,* Van Ness et al, *supra,* whereas special conditions to be taken into consideration are disclosed herein for special embodiments. Generally, concentrations used in the art for recombinases, SBB proteins and/or recombinase-assisting factors used in context of an isothermal amplification, in particular an RPA reaction, can also be used for recombinases, SBB proteins and/or recombinase-assisting factors, respectively, of the present invention. A non-limiting, exemplary composition and procedure of an isothermal amplification using proteins of the present invention is shown in example 5.

For the purpose of the present invention, a short amplicon length will usually be preferable, as it increases the efficiency of the amplification. Further, the shorter the target to be amplified (and thus the shorter the amplicon), the faster the results of the cognate test will be available. Of course, this will represent a huge advantage for rapid tests performed, for example, at home to have rapid certainty on a possible infection.

The design of primers and optionally a probe will be of particular importance when the methods as disclosed herein are performed to provide reliable diagnostic results. In preferred embodiments according to the methods of the present invention, particularly if used for diagnostic purposes, the general amplicon length, which can be influenced by the corresponding primer design, will usually range from about 50 to about 400 base pairs (bp), preferably 100 to about 200 bp, and most preferably about 120 to about 150 bp. An amplicon length below 200 bp is considered to be favorable for diagnostic purposes as the length of the amplicon has an influence on the efficiency of the amplicon amplification and, therefore, is directly linked to the analytical sensitivity of the reaction.

Unlike for PCR, primers in context of the present invention are usually relatively long (between about 30 and 35 nucleotides). Shorter primers (between 18 and 25 nucleotides) can also be used in the present invention but may decrease the reaction speed and sensitivity, which is to be avoided in diagnostic settings.

In one embodiment of the methods of the present invention, at least one forward and at least one reverse primer are provided as target sequence specific primers, wherein at least one of these primers, preferably the reverse primer, is provided at a higher concentration in relation to the other primer, wherein the concentration may, for example, be about 1.1, 1.2, 1.3, 1.4, 1.5, 1.6, 1.7, 1.8, 1.9, 2, 2.1 to 2.5, 2.6 to 2.9, 3 to 4, 5 to 6, 7 to 8 or 9 to 10 times higher. Usually, primers, or a set of primers, or several sets of primers, of a multiplex analysis is of interest, can be provided according to the methods as disclosed herein, wherein the primers are usually provided in an equimolar ration. Still, it was found out that for certain purposes, e.g., for diagnostic of an RNA virus, it may be suitable to provide a higher concentration of one primer (depending on the fact whether it is a (+) or (-) ssRNA virus) to obtain more cDNA for the following amplification step, which will increase the performance of the reaction.

Isothermal amplification was initially demonstrated to be a nucleic acid amplification method for DNA. It was later also shown to be suitable for RNA amplification if an additional enzyme, a reverse transcriptase (e.g., a Murine Leukemia virus (MuLV) reverse transcriptase) is added to the same reaction compartment. In the context of viral diagnostics, the RNA amplification and thereby detection of viral RNA is immensely important. It does not only allow the detection of genetic material from RNA viruses, it further allows the detection of viral transcripts also from DNA viruses. The latter can be of great importance, for example, in such cases where viral transcripts are released into the bloodstream while the virus particles and the viral DNA mostly remain within a tissue from which a biological sample cannot easily be provided.

In embodiments relating to the amplification of RNA, the method may comprise a reverse transcriptase. The method may comprise a protein comprising both the functionality of a DNA polymerase and a reverse transcriptase so that one protein may be used to fulfill both functions.

In certain embodiments, the kits and methods of the present invention may be used for a target sequence that is a single stranded or double stranded DNA, cDNA and/or RNA sequence. In turn, the kits and methods can be used for all kinds of amplification reactions on all kinds of nucleic acid material.

In certain embodiments, DNA and RNA nucleic acid sequences as target sequences are provided. For the detection of DNA viruses or retroviruses the combined detection of both viral DNA and viral RNA (by reverse transcription in the same procedure) offers the great advantage that the sensitivity is strongly increased as all possible targets that may be present in a sample, such as the viral DNA itself and additionally the RNA transcripts of the viral DNA, can be detected together.

In one embodiment, the method is conducted at a temperature of about 20°C to about 50°C, preferably of about 22°C to about 45°C, and most preferably from about 25°C to about 42°C.

A reaction according to the present invention may be incubated between 5 minutes and 16 hours, such as between 15 minutes and 3 hours or between 30 minutes and 2 hours. The incubation may be performed until a desired degree of amplification is achieved (cf. EP 2 336 361 A2). In certain embodiments, the reaction may be performed under agitation to increase the contact rate of the various components and, thus, the efficacy of the method.

In certain embodiments, a probe may be used in the kits and methods as disclosed herein, wherein preferably at least two different probes are used: one associated with the target nucleic acid to be amplified, and one associated with a positive control. The use of reliable positive controls, particularly in the field of diagnosis, more particularly the diagnosis of infections agents like SARS-CoV-2, is very helpful and not included in presently available test kits, as the positive control allows a conclusion whether the reaction as such worked. This is of particular importance and a huge advantage in case the test is not performed by trained experts in a laboratory but rather in a home environment or in test stations.

In the context of the present invention, any luminescent detection system may be used to detect an amplification result of interest fast and reliably. Luminescence is the umbrella term for any kind of spontaneous emission of light by a substance, not resulting from heat. Luminescence comprises various types of luminescence, wherein the most relevant for the purpose of the present invention are chemiluminescence, including bioluminescence and electroluminescence as well as photoluminescence, including fluorescence and phosphorescence.

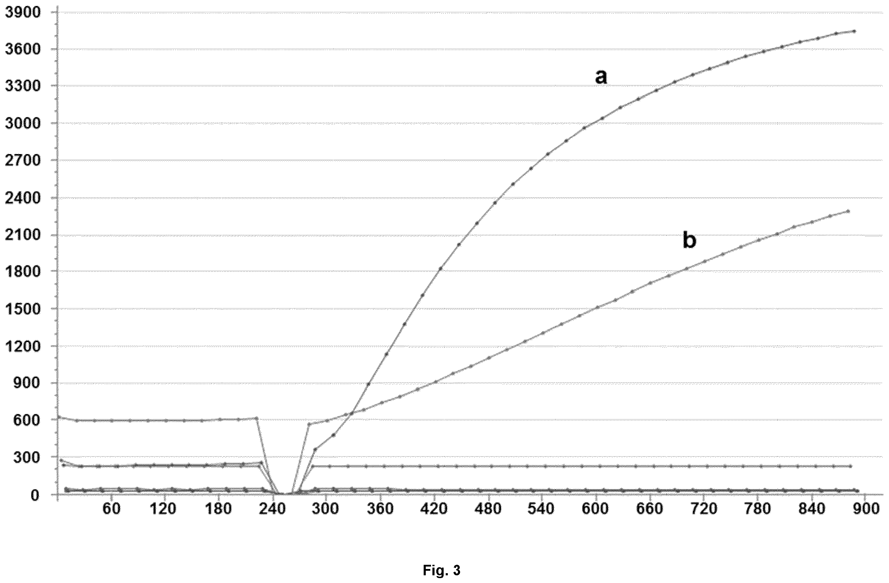

Probes that can be incorporated in the kits and methods according to the present invention include, for example, a commercially available TwistAmp^{™} exo probe (typically having a length of about between 46 and 52 nucleotides) and the TwistAmp^{®} fpg probe(typically between 32 and 35 nucleotides). These probes are used for fluorogenic real-time detection. These two probes are usually labelled with a fluorophore, a quencher (e.g., a BlackHole Quencher) that is in close proximity to the fluorophore to temporarily deter a fluorescent signal, and a blocker (e.g.C3-spacer, a phosphate, a biotin-TEG or an amine) at the 3'-end serving to prevent polymerase extension from the 3'-end (see again Li et al., Analyst, 2019, supra*,* particularly Fig. 3A and B). The real-time detection is based on cleavage of fluorogenic probes at an abasic site (also known as an apurinic/apyrimidinic site that is a location in DNA (less often in RNA), which has neither a purine nor a pyrimidine base) between the fluorophor and the quencher. The abasic site can either be tetrahydrofuran (THF) or a dSpacer (a derivative of the THF) or a dR group (the deoxyribose of the abasic site via a C-O-C linker). The *E. coli* exonuclease III, for example, cleaves the TwistAmp^{™}exo probe at a THF or a dSpacer site, while the *E. coli* glycosylase/lyase fpg (formamidopyrimidine-DNA glycosylase) cleaves the TwistAmp^{™} fpg probe at the dR position. After the enzymatic cleavage, the TwistAmp^{®} exo probe can serve as a forward primer. However, the TwistAmp^{™} fpg probe cannot serve as a primer due to a different catalytic mode (beta-elimination) of the *E. coli* glycosylase/lyase fpg protein, which does not generate an extendable 3'-OH group but a 3'-phosphate group. A variety of different probes or labels for marking a nucleic acid base or a nucleotide of interest with a detectable label, including probes not necessitating the addition of at least one activating enzyme, are imaginable as long as there is at least one suitable light source and a cognate detection device specifically detecting the presence of the probe and/or label by producing a detectable signal.

In another embodiment, a further luminescent luminophore dye may be used (cf. Roy et al., Biosens Bioelectron. 2016 Dec 15; doi: 10.1016/j.bios.2016.06.065.).

As it is known in the field of molecular detection techniques, comprising the use of PCR and of isothermal methods, there are various ways of visualizing the result of an amplification reaction. These techniques are known to the skilled person and can be used for the kits and methods as disclosed herein.

In certain embodiments of the sixths or the eights aspect, the at least one target specific primer, including single-stranded and partially double-stranded primers, is itself labelled with a detectable label. It should be noted that a fluorescence quencher is also considered a detectable label. For example, the fluorescence quencher may be contacted to a fluorescent dye and the amount of quenching is detected. Not to interfere with the reaction, the detectable label should be such that it does not interfere with the amplification reaction. Where the primer is partially double-stranded with an invading strand and a non-invading strand, the detectable label should be attached in such a way so it would not interfere with the amplification reaction of the invading strand. The non-invading strand of a partially double-stranded primer is not elongated so there are no limitations on the labelling of the non-invading strand with the sole exception being that the label on the non-invading strand should not interfere with the elongation reaction of the invading strand. Labelled primers offer the advantage of a more rapid detection of amplified product. In addition, the detection of unincorporated label, that is, labelled oligonucleotides that have not been extended, will allow the monitoring of the status of the reaction.

In another embodiment, a double stranded primer may be labelled as such that the separation of the two strands of the primer may be detected. As discussed above, after multiple rounds of amplification, the invading strand and the non-invading strand of a partially double stranded primer are separated. After this separation, the non-invading strand does not participate in the amplification reaction, which in turn may be used to detect and monitor the result of the amplification reaction on-line.

Further, the detectable label may be a fluorescent label or an enzyme and the label quencher (also referred to as the label inhibitor) may be a fluorescence quencher or an enzyme inhibitor. In these cases, the label is detected by fluorescence or enzyme inhibition. The delectability of the label would be the fluorescence if a fluorescent label is used or enzyme activity if an enzyme is used.

In some embodiments of the sixth or eighth aspect, a separate probe is provided, which may be target sequence specific. Further, the probe may serve the purpose to provide a detectable or visible signal to monitor the result of the amplification reaction in a convenient manner. The probe may carry an activatable label or signal-giving component, which can be activated, for example, by the addition of an enzyme, for example an exonuclease III (Exo III, exo herein) as used in Twist-RPA reactions. The label may also be associated with at least one primer, in certain embodiments. Even though the detectable moiety may be selected from the group consisting of a fluorochrome, an enzyme, a fluorescence quencher, an enzyme inhibitor, a radioactive label, a chromogenic agent, and a combination thereof, for a non-laboratory use of the methods of the present invention, the use of radioactive material and/or generally the use of hazardous substances (of chemical, biological or other nature) should be avoided to allow a fast regulation and safety for the end user of the system, particularly if used in a private environment. Further, this may be favorable for waste management, as the system when used can be easily discarded.

In some embodiments of the sixth or eighth aspect, at least one probe and/or label is provided, wherein the at least one probe and/or the at least one label comprise a directly or indirectly detectable moiety.

All primers or probes as used in the kits and methods as disclosed herein can be naturally occurring DNA and/or RNA sequences, or synthetic primers or probes including naturally occurring bases and/or backbones, or synthetic elements including labels covalently and/or non-covalently attached to the primer or probe, or including at least one phosphothioate backbone for increasing the stability of the primer or probe, or to improve the amplification of DNA sequences by DNA polymerases with proofreading activity (Skerra, Nucleic Acids Res 20, 3551-3554, doi:10.1093/nar/20.14.3551 (1992)), and any combination thereof.

The at least one SSB protein as used according to any embodiment of the present invention may be used in a concentration between 50 ng/µl and 1500 ng/µl, preferably between 100 ng/µl and 800 ng/µl, more preferably between 200 ng/µl and 600 ng/µl and most preferably between 300 ng/µl and 500 ng/µl.

According to the various embodiments of the present invention, every protein may comprise at least one tag, either at the N-terminus, or at the C-terminus, or also within the protein sequence (without disturbing the function of the protein, preferably between to individual domains), or any combination thereof (e.g., N- terminal and C-terminal tag, which will usually be different, e.g. two different affinity tags, or the combination of a solubility tag and a visualization tag etc.).

Alternatively or additionally, nanostructured elements may be used in the reaction mixtures of the sixth or eighth aspect of the present invention to further optimize the amplification result, for example, ZnO nanostructures (Ma et al., Virus Res., 2017, 232: 34-40. doi: 10.1016/j.virusres.2017.01.021.).

The present invention will now be further illustrated with reference to the following non-limiting Examples.

### Examples

### Example 1: Identification of novel recombinase and SSB proteins

In order to identify novel recombinases and SSB proteins, gene and protein sequences having moderate or high sequence similarity to known Recombinase and SSB proteins were searched, as among these the highest likelihood to identify interesting candidates was assumed.

### Bioinformatic identification of novel Recombinases

In a first step, 98 different genomes were searched for candidate genes. Surprisingly, in the majority of genomes, no recombinase was annotated (e.g. *Heliobacter* phages and *Aeromonas* phages). In contrast, some genomes are coding for more than one recombinase (*Escherichia* phages, *Salmonella* phages, *Shigella* phagesoder *Burkholderia* phages). The encoded recombinases were predominantly annotated as NinB7NinG, RecT/E, tyrosine recombinases or YqaJ recombinases. Several further recombinases were not annotated to any family and described as putative recombinases. RecA-like recombinaes were only annotated in two *Arthrobacter* phages. However, these showed very low sequence identity with the known recombinase T6 UvsX recombinase, suggesting a low probability that these are good candidates. Based on the available sequence data, a phylogenetic tree was created. This analysis suggested two candidates: *Pseudomonas* virus H66 recombination-associated protein RdgC und *Bacillus* virus pony RecF recombinase protein. However, sequence alignment with T6 UvsX, again, showed a very low sequence identity.

Based on the phylogenetic analysis, a BLAST (NCBI) amino acid sequence search was conducted and evaluated with T6 UvsX serving as a reference sequence. Among the great number of hits, five different candidates were chosen among the hits with high or moderate sequence identity. Of these five proteins, DNA recombination/repair protein RecA [Lutimaribacter sp. EGI FJ00015] was considered the most promising candidate. Therefore, available sequence data of different *Lutimaribacter* strains was analyzed and compared. Interestingly, sp. EGI FJ00015 encodes two different RecA sequences - herein referred to as RecA1 and RecA2. RecA2 is a classical RecA homolog found in all available *Lutimaribacter* genomes, whereas RecA1 is similar to UvsX enzymes. Still, RecA1 and RecA2 have a high sequence similarity among each other.

The genome analyses of different *Lutimaribacter* strains suggest that bacterial UvsX-like recombinases could promising candidates. Thus, a new BLAST search was conducted, limited to bacterial taxa. Among the resulting hits, RecA1 from *Lutimaribacter* sp. EGI FJ00015 (high similarity to known T6 UvsX) and RecA from *Shigella flexneri* (moderately similar to T6 UvsX) chosen for structural analysis.

### Structural analysis of novel recombinase variants

As no structural information was available for the new protiens, 2D elements were predicted using the program Psi Pred and a 2D structural alignment was created using the software 2dss. Finally, a 3D structure was created using AlphaFold. The resulting structures were compared with the structures of T4 UvsX and T4 Gp2, obtained by x-ray crystallography. Both the 2D and the 3D structural analysis indicated a high degree of similarity up to approx. amino acid 340 for the two newly identified recombinase variants, followed by stronger differences in the predicted structure.

### Identification of novel SSB proteins

Using BLAST, bacterial SSB proteins were searched, with RB69 GP32 serving as reference sequence. Within the group of very similar proteins, was a protein annotated as hypothetical protein from *Lutimaribacter* sp. EGI FJ00015 and a protein from *Shigella flexneri* annotated as single-stranded DNA-binding protein were identified. Both showed a high sequence similarity with RB69 Gp32 and both candidates were chosen for further structural analysis.

### Structural analysis for novel SSB variants

2D Structural elements showed a very high similarity with Rb69 Gp32 approx. until amino acid 220, followed by stronger structural differences. Smaller deviations were also identified between amino acid 30 and 80. Analysis of the 3D structure confirmed these results and showed a high similarity approx. until amino acid 235.

### Example 2: Oligonucleotide binding analysis

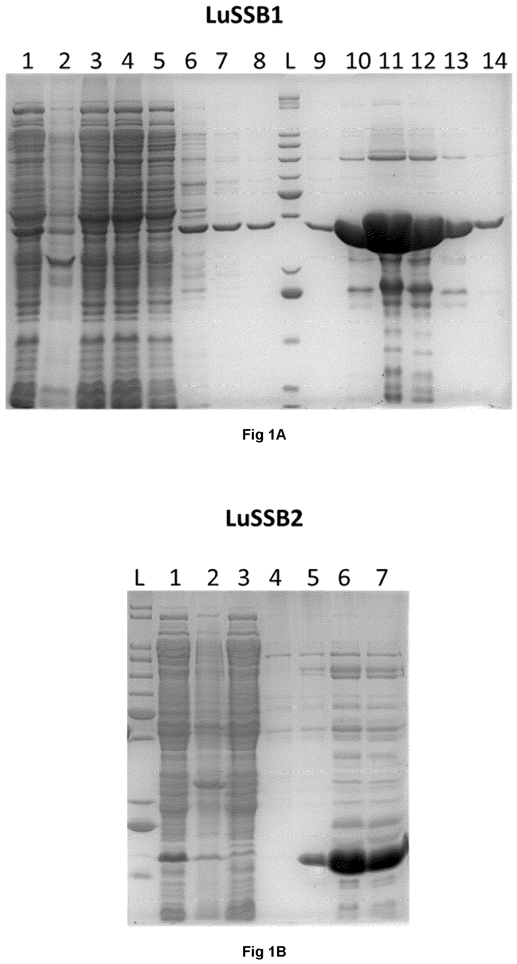

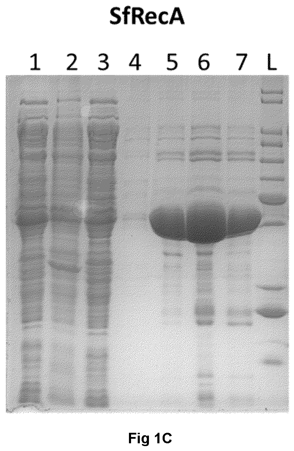

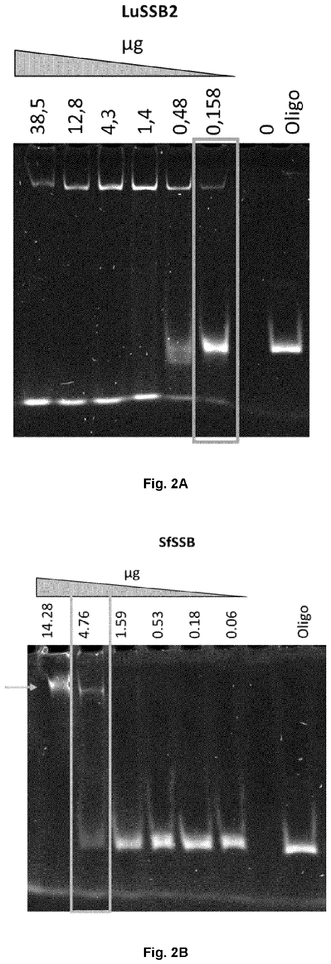

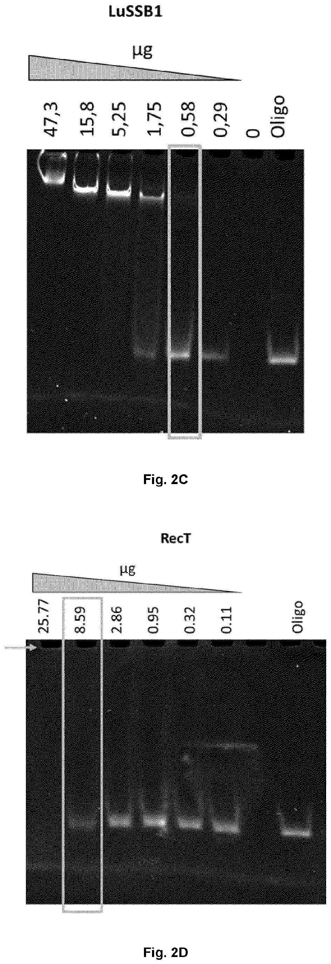

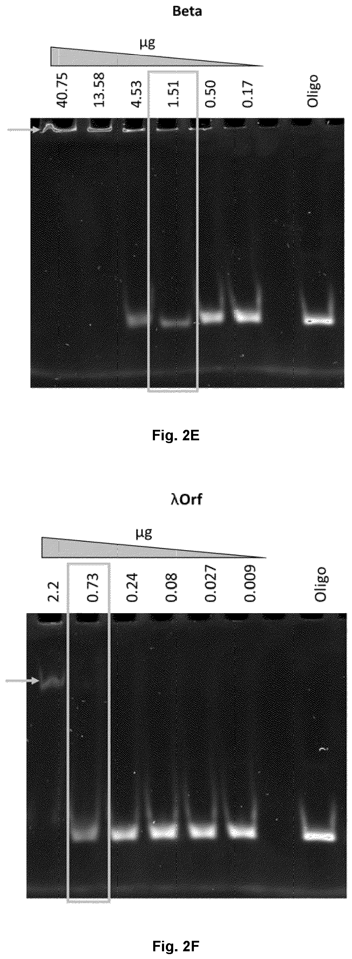

**Figure 1A** to 1C show example purifications of LuSSB1, LuSSB1 and SfRecA. To test to Oligonucleotide binding activity of the purified proteins, electrophoretic mobility shift assays (EMSA) were performed for multiple newly identified variant (see **Fig. 2A** to **2F** and **table 1**).

The assays were based on the detection of a gel shift of a FAM-labelled oligonucleotide after protein binding. The limit for shifting the oligonucleotide is determined using polyacrylamide gels. For the binding reaction RPA conditions are applied. Reaction conditions for oligonucleotide binding in 20 µL reactions: 20 mM tris-acetate pH 7.4, 90 mM KOAc, 10 mM MgOAc, 2 mM DTT, 1.23 µM oligonucleotide and varying amounts of protein (dilution series in 20mM tris-acetate pH 7.4). The reaction was started by adding the protein to the incubation mix and let run for 15 min at 42°C. After 15 min, the reaction was stopped by adding 20 µL pre-cooled 40% glycerol.

After incubation, samples were run in a 12% TBE gel and the FAM label of the oligonucleotide was detected.

**Table 1 Results of the EMSA**

| **Protein** | **Minimal concentration for shift [µg]** |
|---|---|
| LuSSB2 | 0.158 |
| SfSSB | 4,76 |
| LuSSB1 | 0.58 |
| RecT | 8,95 |
| Beta | 1,51 |
| Orf | 0,73 |
| T4 Gp32 (BR) | 0,43 |
| Rb69 Gp32 (BR) | 0,66 |
| T4 Gp32 (NEB) | 2,7 |

### Example 3: Activity assay for Recombinase and recombinase-assisting factor

Recombinase activity of purified proteins was tested based on the conversion of ATP to ADP by the recombinase

Reaction mixtures of 20 µl contained, 50 mM tris-acetate/KOAc pH 7.9, 150 mM KOAc, 14 mM MgOAc, 5 mM DTT, 2mM ATP, as well as varying amounts of ssDNA and recombinase protein. Reactions were started at 42 °C by adding the MgOAc and incubated to 2, 4 and 8 minutes and stopped by 1 to 36 dilution in water an immediate freezing. A negative control contains the same components but is not started by adding MgOAc. Samples and control were then analyzed using the Kinase-Glo^{®} Max assay a Kinase-Glo^{®} Max assay in which a luminescence signal generated by a luciferase correlates linearly with the ATP concentration in the sample.

Exemplary results of activity measurements are:

| **SfRecA** | **Control** - **UvsX** |
|---|---|
| 4,95 U/mg | 4,1 U/mg |

The Activity of recombinase-assisting factors was analyzed in a similar manner. A recombinase-assisting factor does not convert ATP to ADP, however, under specific conditions (K-acetate concentrations >150 mM) it is able to increase the activity of a recombinase. In this assay, the concentration of recombinase-assisting factor is determined, which leads to a doubling of the recombinase activity. Reaction mixtures are the same as above, except that also a recombinase-assisting factor is added.

### Example 5: Isothermal amplification with novel proteins

A variety of different combinations and conditions were set up to test the newly identified recombinase and SSB proteins in isothermal amplification reactions. Exemplary results for LuSSB1 are shown in **Fig. 3****.**

RPA reactions were set up in 50 µl samples with the following protein composition:

| **Reference** | | **LuSSB1** | |
|---|---|---|---|
| Protein | ng/µl | Protein | ng/µl |
| UvsX | 300 | | 300 |
| UvsY | 50 | | 50 |
| Rb69 Gp32 | 560 | LuSSB1 | 150 |
| Creatin-kinase (CK) | 50 | | 50 |
| *Bsu* polymerase | 96 | | 96 |
| Exonuclease | 80 | | 80 |

RPA reactions were run in an Axxin T8 Isothermal Instrument.

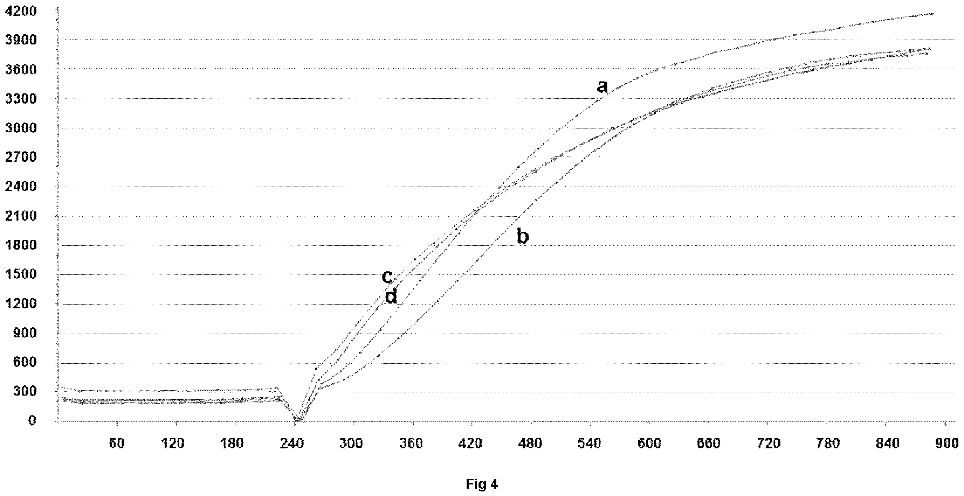

**Figure 4** shows further exemplary RPA results of using either 330 ng/µl LuSSB1 or 580ng/µl Rb69 GP32 in reaction mixes as shown above.

Further reaction mixes were tested as shown above with either 400 ng/µl LuSSB1 or 600 ng/µl Rb69 Gp32.

Interestingly LuSSB1 showed a highly increased performance compared to RB69 Gp32 in an otherwise identical RPA reaction. Therefore, LuSSB1 poses a considerable enhancement to standard RPA reaction. Different amplification set-ups with LuSSB1 as well as the other new variants of the present disclosure have been conducted and further experiments are ongoing.

### Example 6: Asymmetric primer concentrations

To achieve optimal amplification results, the proteins of the present amplification can be used in amplification mixtures comprising asymmetric primer concentrations. The provision of one primer, preferably the reverse primer, of a primer pair in a higher concentration can boost the amplification reaction, especially for lower amounts of target nucleic acid to be detected.

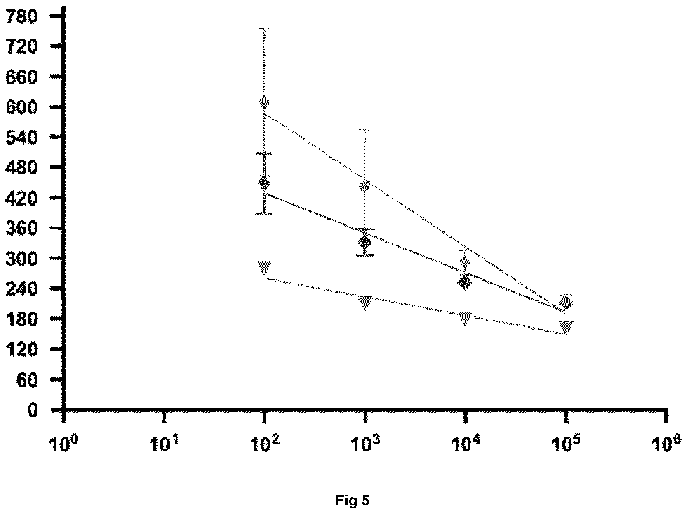

Exemplary amplification reactions with symmetric and asymmetric primer concentrations for the amplification of *Monkeypox virus* DNA are shown in **Fig. 5** using the following reaction mixes:

| **Mix 1** | | **Mix 2** | |
|---|---|---|---|
| Protein | ng/µl | Protein | ng/µl |
| UvsX | 120 | UvsX | 310 |
| UvsY | 60 | UvsY | 50 |
| T4 Gp32 | 600 | RB 69 Gp32 | 600 |
| Creatin-kinase | 100 | Creatin-kinase | 50 |
| *Bsu* polymerase | 30 | *Bsu* polymerase | 30 |
| Exonuclease | 80 | Exonuclease | 80 |

Primer concentrations were either symmetric (both primers as 210 nM) or asymmetric (forward primer: 210 mM; reverse primer 840 mM).

Analyses with the proteins of the present invention in reaction mixtures with asymmetric primer pairs are ongoing and suggest that such approaches, using the novel enzymes of the invention in combination with asymmetric primer concentrations, could pose a substantial improvement over currently available amplification methods.

## Claims

1. A protein, or a functional fragment thereof, preferably supporting isothermal amplification, wherein the protein comprises or consists of:
a) a *Lutimaribacter* SSB1 comprising or consisting of the amino acid sequence of SEQ ID NO: 1, or a sequence having at least 90%, 91%, 92%, 93%, 94%, 95%, 96%, 97%, 98% or at least 99% sequence identity thereto, preferably wherein the amino acid sequence has a serine residue at position 64 according to SEQ ID NO 1 or 2; or
b) a *Lutimaribacter* SSB2 comprising or consisting of the amino acid sequence of SEQ ID NO: 2, or a sequence having at least 90%, 91%, 92%, 93%, 94%, 95%, 96%, 97%, 98% or at least 99% sequence identity thereto; or
c) a *Shigella flexneri* SSB comprising or consisting of the amino acid sequence of SEQ ID NO: 3, or a sequence having at least 90%, 91%, 92%, 93%, 94%, 95%, 96%, 97%, 98% or at least 99% sequence identity thereto; or
d) an Bacteriophage A Beta protein comprising or consisting of the amino acid sequence of SEQ ID NO: 4, or a sequence having at least 90%, 91%, 92%, 93%, 94%, 95%, 96%, 97%, 98% or at least 99% sequence identity thereto; or
e) a *Lutimaribacter* RecA1 comprising or consisting of the amino acid sequence of SEQ ID NO: 5 or 6, or a sequence having at least 90%, 91%, 92%, 93%, 94%, 95%, 96%, 97%, 98% or at least 99% sequence identity thereto; or
f) a *Lutimaribacter* RecA2 comprising or consisting of the amino acid sequence of SEQ ID NO: 7, or a sequence having at least 90%, 91%, 92%, 93%, 94%, 95%, 96%, 97%, 98% or at least 99% sequence identity thereto; or
g) a *Shigella flexneri* RecA comprising or consisting of the amino acid sequence of SEQ ID NO: 8, or a sequence having at least 90%, 91%, 92%, 93%, 94%, 95%, 96%, 97%, 98% or at least 99% sequence identity thereto; or
h) a *Escherichia coli* RecT comprising or consisting of the amino acid sequence of SEQ ID NO: 9, or a sequence having at least 90%, 91%, 92%, 93%, 94%, 95%, 96%, 97%, 98% or at least 99% sequence identity thereto; or
i) a Bacteriophage A Orf comprising or consisting of the amino acid sequence of SEQ ID NO: 10, or a sequence having at least 90%, 91%, 92%, 93%, 94%, 95%, 96%, 97%, 98% or at least 99% sequence identity thereto.

2. A fusion protein comprising at least one protein of claim 1 and at least one further functional portion, the at least two portions optionally being separated by at least one linker.

3. The fusion protein of claim 2, wherein the fusion protein comprises at least one affinity tag, preferably at the C-terminus and/or N-terminus.

4. The fusion protein of claim 2, wherein the fusion protein comprises at least one GST-tag, MBP-tag, Strep-tag and/or HIS-tag, preferably comprising at least one HIS-tag.

5. A nucleic acid molecule encoding at least protein of claim 1; and/or at least one fusion protein of any one of claims 2 to 4.

6. A vector or expression construct comprising at least one nucleic acid molecule of claim 5.

7. A cell comprising at least one protein of claim 1; and/or at least one fusion protein of any one of claims 2 to 4; and/or at least one nucleic acid molecule of claim 5; and/or at least one vector or expression construct of claim 6.

8. A kit comprising at least one protein of claim 1; and/or at least one fusion protein of any one of claims 2 to 4; and/or at least one nucleic acid molecule of claim 5; and/or at least one vector or expression construct of claim 6; and/or at least one cell of claim 7.

9. The kit of claim 8, wherein the kit comprises
(i) at least one single-stranded binding protein, optionally of claim 1 a), b), c) and/or d), preferably of claim 1 a), and/or at least fusion protein of any one of claims 2 to 4 comprising the same;
(ii) at least on recombinase, optionally of claim 1 e), f), g) and/or h), and/or at least fusion protein of any one of claims 2 to 4 comprising the same;
(iii) optionally at least one recombinase-assisting factor, optionally of claim 1 i), and/or at least fusion protein of any one of claims 2 to 4 comprising the same;
(iv) at least one, preferably at least two, target sequence specific primers;
(v) at least one DNA polymerase;
(vi) optionally at least one reverse transcriptase;
(vii) suitable reaction components, including at least one suitable buffer, dNTPs or a mixture of dNTPs and ddNTPs, optionally ATP or an ATP analog, and further optionally a crowding agent;
(viii) optionally at least one probe and further optionally at least one enzyme activating the probe.

10. A use of protein of claim 1; and/or a fusion protein of any one of claims 2 to 4; and/or a nucleic acid molecule of claim 5; and/or a vector or expression construct of claim 6; a cell of claim 7, and/or a kit of claim 8 or 9 for isothermal amplification of at least one target nucleic acid molecule, optionally for detection of viral RNA and/or DNA, and/or for sample amplification for nucleic acid sequencing, preferably wherein the amplification is performed *in vitro* and/or preferably, wherein the amplification is performed as *in situ* available test, preferably using a kit of claim 8 or claim 9.

11. An isothermal method of nucleic acid amplification of at least one target sequence, preferably in a biological sample, the method comprising the steps of:
(a) providing
(i) at least one single-stranded binding protein, optionally of claim 1 a), b), c) and/or d), preferably of claim 1 a), and/or at least fusion protein of any one of claims 2 to 4 comprising the same;
(ii) at least on recombinase, optionally of claim 1 e), f), g) and/or h), and/or at least fusion protein of any one of claims 2 to 4 comprising the same;
(iii) optionally at least one recombinase-assisting factor, optionally of claim 1 i), and/or at least fusion protein of any one of claims 2 to 4 comprising the same;
(iv) at least one, preferably at least two, target sequence specific primers;
(v) at least one DNA polymerase;
(vi) optionally at least one reverse transcriptase;
(vii) suitable reaction components, including at least one suitable buffer, dNTPs or a mixture of dNTPs and ddNTPs, optionally ATP or an ATP analog and further optionally a crowding agent;
(b) providing at least one biological sample to be analyzed for the presence of the at least one target sequence and optionally providing a preferably sterile extraction kit to obtain the biological sample;
(c) optionally: providing at least one probe and optionally providing at least one enzyme activating the probe so that a detectable signal is generated;
(d) adding a starting reagent to initiate the amplification reaction; and
(e) obtaining the at least one amplified target sequence and/or obtaining at least one detectable signal each signal being indicative of a successful amplification of the respective target sequence amplified,
wherein step (a) comprises in sub-step (i), (ii) and/or (ii) the provision of at least one protein of claim 1 and/or at least one fusion protein of any one of claims 2 to 4.

12. The method of claim 11, wherein the method does not comprise the use of ATP or an ATP analog and/or an energy regeneration system.

13. The method of claim 11 or 12. wherein the method is performed absent a recombinase-assisting factor.
